(19)

Europäisches
Patentamt
European
Patent Office
Office européen
des brevets

(11) **EP 1 837 651 A1**

(12) **EUROPEAN PATENT APPLICATION**

(43) Date of publication:
26.09.2007 Bulletin 2007/39

(51) Int Cl.:
*G01N 31/10* (2006.01)     *C40B 40/18* (2006.01)

(21) Application number: 06110693.6

(22) Date of filing: 06.03.2006

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Degussa GmbH**
**40474 Düsseldorf (DE)**

(72) Inventors:
• **Wolf, Dorit Dr.**
**61440 Oberursel (DE)**

• **Tacke, Thomas Dr.**
**63755 Alzenau (DE)**
• **Seebald, Steffen Dr.**
**63796 Kahl am Rhein (DE)**

(74) Representative: **Weber, Wolfgang et al**
**Degussa GmbH**
**Intellectual Property Management**
**Patente und Marken**
**Standort Hanau**
**Postfach 13 45**
**63403 Hanau (DE)**

(54) **Evaluation of the performance profile of catalysts**

(57)    Library of catalysts, whereby each catalyst is defined by a specific performance profile. The application also relates to a method to produce the library and a method to elect a catalyst. A catalytic performance profiling has been developed with which catalytic characteristics of catalysts can be elucidated. The catalytic profiling analysis includes measuring activity and selectivity values for a set of test reactions and deriving performance profiles. These performance profiles allow a statistical analysis of similarities.

b)

**Figure 1:** Illustration of the concept for catalyst profiling based on a set of sensitive test reactions:

a) Determination of particular performance data for an individual catalyst (activity and selectivity values in different test reactions)
b) Visualization of performance profiles from the particular performance values as fingerprints for individual catalysts as a basis of similarity analysis

**Description**

The subject of the invention is a library of catalysts, a method to produce the library and a method to elect a catalyst.

The development of heterogeneous catalysts is related to the challenge that solid properties determining the catalytic properties are not easily accessible. This is especially true for fine tuning of selectivity and long-term catalytic stability where gradual changes by 1 % are already of importance. Regardless the fact that catalysts do not show obvious differences with respect to solid properties (e. g. metal particle size, particle dispersion or solid phase and oxidation state of the active metal) they often reveal differences in their catalytic behaviour. An additional challenge in catalyst development is related to the complex dependency between solid properties themselves. This may lead to the situation that particular properties cannot be changed independently without influencing others being also important for catalytic performance (e. g., the simultaneous change of metal particle size together with the change of their radial distribution on the support or the simultaneous change of metal oxidation state together with the change of dispersion etc.)

For industrial application of catalysts in fine chemistry these circumstances are serious obstacles for a straightforward rational development and the identification of suitable catalysts for conversion of certain substrates.

Therefore there was the problem to find a method to find a catalyst showing the performance profile promising the best effort.

Subject of the invention is a catalyst, characterized in that, it is defined by a specific performance profile.

In a preferred subject of the invention the catalyst is a heterogeneous catalyst.

The performance profile of the catalyst can be estimated, whereby the catalyst is first synthesized and then used in at least two different chemical reactions assigned to various chemical reaction classes comprising preferentially

hydrogenation of carbonyl compounds

hydrogenation of olefins or polyolefins

hydrogenation of aromatics or heteroaromatics

hydrogenation of nitro-compounds

hydrogenation of nitriles,

hydrogenation of imines,

hydrogenation of hydroxylamines,

hydrogenation of alkynes,

reductive alkylation of primary or secondary amines,

reductive amination of aldehydes or ketones by ammonia salts or by amines,

hydrogenolysis of C-C bonds, ethers, carbamates, carbonates, amines or organic sulfides,

hydrodehalogenation of halo-aromatics or halo-aliphatics,

dehydrogenation of cycloalkanes or cycloalkenes, isomerization of hydroxy-olefins,

hydrogenation of multifunctional substrates including at least two of the following functional groups or structural units:

CC-double bond CC- triple bond, nitro-, alcohol-, carbonyl-, carboxyl-, nitril-, imine, hydroxylamine-, azo-, diazo-, halogen-, ether-group, aromatic rings,

oxidation of alcoholes,

oxidation of aldehydes,

oxidation of olefins,

oxidation of multifunctional substrates including at least two of the following functional groups:

CC-double bond, CC triple bond, alcohol-, carbonyl-, nitril-, imine, hydroxylamine-, azo-, diazo-group,

C-C coupling,

enantioselective hydrogenation of carbonyl compounds, enantioselective reductive alkylation of primary or secondary amines,

enantioselective reductive amination of aldehydes or ketones by ammonia salts or by amines,

whereby in respect to each reaction the catalyst performance is estimated and correlated to a defined table in order to set up the performance profile.

The defined table for the correlation of the performance of the catalyst may be a table, in which the different reaction typs are noted in a specific sequence.

In a preferred subject of the invention the sequences of the reaction typs, which is noted in the specific sequence in the table, can be chosen from at least two different

reaction typs selected from the group

hydrogenation of carbonyl compounds

hydrogenation of olefins or polyolefins

hydrogenation of aromatics or heteroaromatics

hydrogenation of nitro-compounds

hydrogenation of nitriles,

hydrogenation of imines,

hydrogenation of hydroxylamines,

hydrogenation of alkynes,

reductive alkylation of primary or secondary amines, reductive amination of aldehydes or ketones by ammonia salts or by amines,

hydrogenolysis of C-C bonds, ethers, carbamates, carbonates, amines or organic sulfides,

hydrodehalogenation of halo-aromatics or halo-aliphatics,

dehydrogenation of cycloalkanes or cycloalkenes, isomerization of hydroxy-olefins,

hydrogenation of multifunctional substrates including at least two of the following functional groups or structural units: CC-double bond CC- triple bond, nitro-, alcohol-, carbonyl-, carboxyl-, nitril-, imine, hydroxylamine-, azo-, diazo-, halogen-, ether-group, aromatic rings,

oxidation of alcoholes,

oxidation of aldehydes,

oxidation of olefins,

oxidation of multifunctional substrates including at least two of the following functional groups:

CC-double bond, CC triple bond, alcohol-, carbonyl -, nitril-, imine, hydroxylamine-, azo-, diazo-group,

C-C coupling,

enantioselective hydrogenation of carbonyl compounds, enantioselective reductive alkylation of primary or secondary amines,

enantioselective reductive amination of aldehydes or ketones by ammonia salts or by amines.

A further subject of the invention is a library of catalysts, characterised in, that each catalyst is defined by a specific performance profile.

In a preferred form of the invention the library can be searched by using an algorithm of the statistical similarity analysis.

The library can consist of homogeneous and/or heterogeneous catalysts. Preferred are heterogeneous catalysts.

A further subject of the invention is a method to produce the library of catalysts, characterized in, that each catalyst is synthesized separately and then used in at least two different chemical reactions assigned to various chemical reaction classes comprising preferentially hydrogenation of carbonyl compounds,

hydrogenation of olefins or polyolefins,

hydrogenation of aromatics and heteroaromatics,

hydrogenation of nitro-compounds,

hydrogenation of nitriles,

hydrogenation of imines,

hydrogenation of hydroxylamines,

hydrogenation of alkynes,

reductive alkylation of primary or secondary amines,

reductive amination of aldehydes or ketones by ammonia salts or by amines,

hydrogenolysis of C-C bonds, carbamates, carbonates, ethers, amines or organic sulfides,

hydrodehalogenation of haloaromatics or haloaliphatics,

dehydrogenation of cycloalkanes or cycloalkenes, isomerization of hydroxy-olefins,

hydrogenation of multifunctional substrates including at least two of the following functional groups or structural units: CC-double bond CC-triple bond, nitro-, alcohol-, carbonyl-, carboxyl-, nitril-, imine, hydroxylamine-, azo-, diazo-, halogen-, ether-group, aromatic rings,

oxidation of alcohols,

oxidation of aldehydes,

oxidation of olefins,

oxidation of multifunctional substrates including at least two of the following functional groups:

CC-double bond, CC-triple bond, alcohol-, carbonyl-, nitril-, imine, hydroxylamine-, azo-, diazo-group,

C-C coupling,

enantioselective hydrogenation of carbonyl compounds, enantioselective reductive alkylation of primary or secondary amines,

enantioselective reductive amination of aldehydes or ketones by ammonia salts or by amines,

whereby in respect each reaction the catalyst performance is estimated and correlated to a defined table in order to set up the performance profile, further on the catalyst is put into the library.

A further subject of the invention is a method to elect a catalyst from the library in respect to a given substrate, which is characterised in, that the substrate, which should be treated with the catalyst, can produce a plurality of compounds, whereby one specific compound is wanted to be produced selectively, whereby the substrate shows a specific profile in respect to the performance of the catalyst needed and this performance profile is compared to the performance profiles of the library according to the invention.

This election can be performed by using algorithm of the statistical similarity analysis.

The substrat according to the invention can be any chemical compound, which owns the structur and/or reactive groups that can undertake at least one reaction assigned to various chemical reaction classes comprising preferentially

hydrogenation of carbonyl compounds

hydrogenation of olefins or polyolefins

hydrogenation of aromatics or heteroaromatics

hydrogenation of nitro-compounds

hydrogenation of nitriles,

hydrogenation of imines,

hydrogenation of hydroxylamines,

hydrogenation of alkynes,

reductive alkylation of primary or secondary amines,

reductive amination of aldehydes or ketones by ammonia salts or by amines,

hydrogenolysis of C-C bonds, ethers, carbamates, carbonates, amines or organic sulfides,

hydrodehalogenation of halo-aromatics or halo-aliphatics,

dehydrogenation of cycloalkanes or cycloalkenes, isomerization of hydroxy-olefins,

hydrogenation of multifunctional substrates including at least two of the following functional groups or structural units: CC-double bond CC- triple bond, nitro-, alcohol-, carbonyl-, carboxyl-, nitril-, imine, hydroxylamine-, azo-, diazo-, halogen-, ether-group, aromatic rings,

oxidation of alcoholes,

oxidation of aldehydes,

oxidation of olefins,

oxidation of multifunctional substrates including at least two of the following functional groups: CC-double bond, CC triple bond, alcohol-, carbonyl -, nitril-, imine, hydroxylamine-, azo-, diazo-group, C-C coupling, enantioselective hydrogenation of carbonyl compounds, enantioselective reductive alkylation of primary or secondary amines,

enantioselective reductive amination of aldehydes or ketones by ammonia salts or by amines.

According to the invention the method (so called "catalytic performance profiling") was developed with which catalytic characteristics of heterogeneous catalysts for fine chemical application can be efficiently and comprehensively elucidated. Moreover, those physical properties can be identified, which do influence the catalytic performance profiles significantly. Thus, the profiling method according to the invention takes into account the complex relationship of physico-chemical parameters of solids and catalytic performance parameters

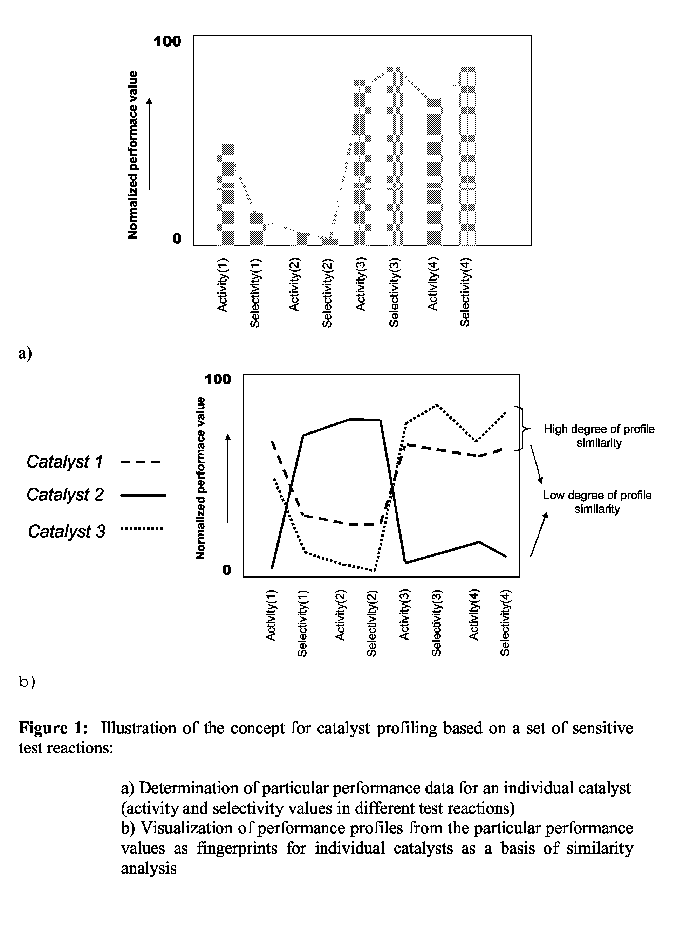

In Figure 1 a) and b) the principles of catalytic profiling analysis according to the invention are explained:

Catalytic profiling analysis includes a set of test reactions which are very sensitive with respect to catalyst properties and/or the recipes of preparation. These test reactions are numbered by 1, 2, 3 and 4.

From activity and selectivity values measured for test reactions 1, 2, 3 and 4 (Fig. 1a) corresponding performance profiles (Fig. 1b)) can be derived which can be understood as catalytic fingerprints for individual catalysts 1, 2 and 3. Thus, performance profiles allow a statistical analysis of similarities (Fig. 1b).

At this point it has to be emphasized that for statistical similarity analysis a large population of catalysts is necessary in order to cover a sufficient number and scale of performance parameters (activities, selectivities in the different reactions). The scale of performance values is a relative one. I. e. the maximum value of a particular performance value in the test population was set to 100 %, while the minimum value was set to 0. Thus, the scale is rather flexible and will certainly change with increasing the test population of catalysts.

A procedure similar to the catalytic performance profiling is applied to profiling of solids' characteristics. Hereby, measurable solid properties (e. g. metal particle size, metal dispersion, binding energies of elements of noble metals or oxygen, pore size distribution of supports etc.) are summarized in solids' characteristics profiles followed by statistical similarity analysis.

According to the state of the art heterogeneous catalysts are developed for certain application by correlating the catalytic performance in a certain reaction with parameters of catalyst composition and preparation as well as by characterizing physico-chemical properties of the catalyst and correlates them with parameters of preparation and catalytic performance (so called knowledge-based rational approach). I. e. a certain catalyst will be optimised for a certain reaction and a certain substrate.

In contrast, the catalytic profiling method according to the invention is a heuristic method which takes into account for the complexity of the relationship between catalyst preparation method and catalytic performance in a large diversity of classes of reactions of (multi-functional) substrates. For this purpose catalytic tests with a variety of reactions are performed. The particular performance values of the catalytic reactions are summarized by the catalytic performance profiles. This approach leads to a unique fingerprint for individual catalysts allowing a fast identification of strength and weaknesses of a catalyst.

Based on this libraries of heterogeneous catalysts according to the invention are built up, which cover a wide range of fine-chemical application of solid catalysts from which suitable catalysts can be chosen rapidly.

Based on the profiling method according to the invention catalysts are unambiguously characterized. Based on the profiling analysis according to the invention catalyst preparation methods can be rapidly optimised with respect to production costs (substitution of complicated preparation methods by easier ones, substitution of expensive raw material by cheaper ones, fast scale-up of catalyst production methods up to technical scale ...).

Based on profiling analysis according to the invention the preferred field of catalyst application (class of reaction and particular substrate) can be much faster identified than with the conventional approach of catalyst development for single reactions. This leads to a significant acceleration of development of heterogeneous catalytic processes.

## Example 1

This example demonstrates that the characterization and optimisation of catalysts for fine-chemical applications, based on catalytic tests is much more informative and more efficient than characterizing physico-chemical properties of catalysts and correlating them with catalytic properties as done for development of heterogeneous catalysts according to the state of the art.

The profiling methodology was validated for four Pd (5 wt-%)/C powder catalysts, prepared by different methods and showing different metal particle size, metal dispersion and oxidation state of Pd. All catalysts were based on the same activated carbon support. Hence, support properties were neglected in the analysis.

For catalytic profiling analysis four different hydrogenation reactions were considered. These included hydrogenation of Cl-nitrobenzene, dibenzylether, cinnamic acid and selective hydrogenation of a 1-hydroxy-3,4-olefin. From these four test reactions the following twelve catalytic performance criteria were derived as basis of catalytic performance profiles:

### Hydrogenation of Cl-nitrobenzene at 10 bars and 25°C

(1) Activity for hydrogen conversion at reaction t = 0
(2) Selectivity with respect to Aniline at complete conversion of substrate
(3) Selectivity with respect to Cl-Aniline at complete conversion of substrate

### Hydrogenation of dibenzylether at 10 bars and 25°C

(6) Activity for hydrogen conversion at reaction time = 0
(7) Activity for hydrogen consumption at reaction time t = 80 min
(8) Total hydrogen consumption at reaction time t = 80 min

### Hydrogenation of cinnamic acid at 10 bars and 25°C

(9) Activity of hydrogen consumption at reaction time t = 0

### Hydrogenation of a 1-hydroxy-3,4-olefin at 10 bars and 70°C

(10) Selectivity to the hydroxy alkane at complete conversion of substrate
(11) Selectivity to the ketone at complete conversion of substrate (formed by isomerization)
(12) Selectivity to the alkane at complete conversion of substrate (formed by double bond hydrogenation and hydrogenolysis of OH group)

For physical characteristics profiles the following eightteen values derived from statistical analysis of TEM data and XPS analysis were used:

(1) asymmetry of particle size distribution referring to metal particle number
(2) asymmetry of particle size distribution referring to metal particle volume
(3) inconsistency of particle size distribution referring to metal particle number
(4) inconsistency of particle size distribution referring to metal particle volume
(5) kurtosis of particle size distribution referring to metal particle number
(6) kurtosis of particle size distribution referring to metal particle volume
(7) mean volume of metal particles / $nm^3$
(8) metal particle size distribution referring to metal particle number / nm
(9) particle size distribution referring to metal particle volume / nm
(10) relative standard deviation of metal particle size distribution referring to metal particle number / %
(11) relative standard deviation of metal particle size distribution referring to volume / %
(12) specific surface area of metal particles / $m^2\ cm^{-3}$
(13) standard deviation of metal particle size distribution referring to metal particle number / nm
(14) standard deviation of metal particle size distribution referring to metal particle volume / nm
(15) XPS Binding energy derived from the Pd 3d 5/2 signal
(16) XPS Binding energy of derived from the O 1s signal
(17) surface atom fraction of palladium (derived from XPS analysis)
(18) surface atom fraction of oxygen (derived from XPS analysis)

For the experimental tests a eightfold batch reactor system (reactor volume 20 ml) with magnetic stirring, which allows the measurement of hydrogen uptake at constant hydrogen pressure was used. Analysis of substrates and products was performed off-line by GC for determining selectivity values. Activity values were derived from hydrogen up-take within a defined time interval.

Fig. 2 shows the complete catalytic performance profiles for the four different catalysts;
Fig. 3 indicates the profiles of physical characteristics. (The sequence of profiling parameters from left to right corresponds to that mentioned above.)

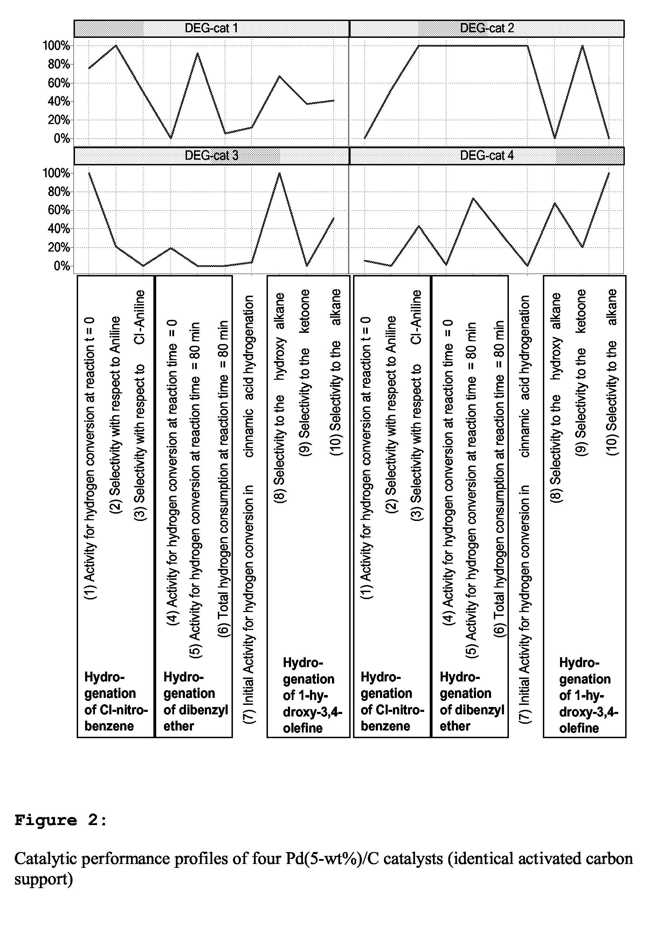

The catalytic performance profiles of the four Pd/C catalysts look rather different (Fig. 2). Similarities are not obvious despite the catalysts have the same Pd loading (5 wt-%) and the same activated carbon support. Thus, the differences in the catalytic behaviour are exclusively determined by the different modes of preparation.

The strongest differences in catalytic behaviour can be derived for samples DEG-cat 2 and DEG-cat 3. While DEG-cat 2 is highly active for debenzylation, cinnamic acid formation and selective for formation of Cl-aniline, DEG-cat 3 shows low activity for debenzylation and cinnamic acid formation as well as low selectivity in Cl-aniline formation. DEG-cat 1 and DEG-cat 4 are in-between of the catalytic performance profiles of DEG-cat 2 and DEG-cat 3.

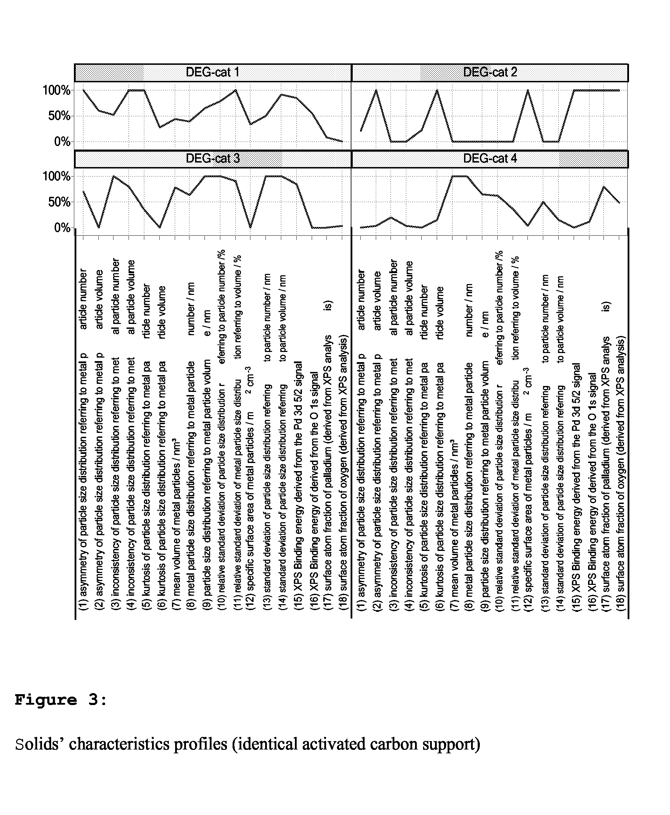

In contrast to the catalytic performance profiles the comparison of profiles of solids' characteristics indicates similarities for DEG-cat 1 and DEG-cat 3 while for DEG-cat 2 and DEG-cat 4 no obvious similarities can be derived (Fig. 3).

In order to rationalize the comparison of profiles a statistical similarity analysis was performed based on Pearson Product Momentum Correlation where profiles with identical shape have maximum correlation and perfectly mirrored profiles have minimum correlation [Hair, J.F.Jr., Anderson, R.E., Tatham, R.L., Black, W.C. (1995) Multi-variate Data Analysis, Fourth Edition, Prentice Hall, Englewood Cliffs, New Jersey].

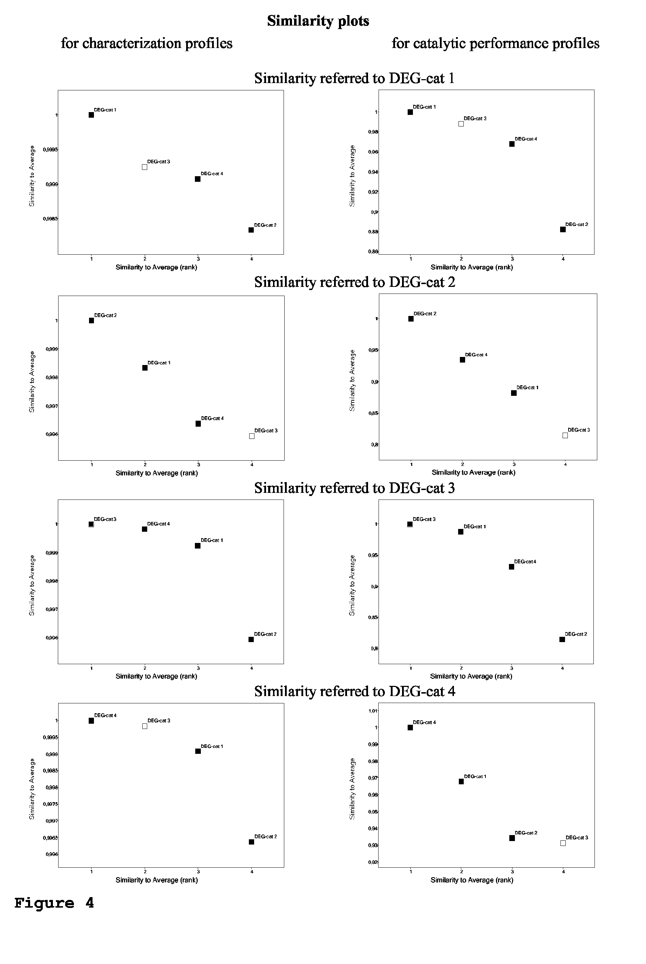

In Fig. 4 the results of statistical similarity analysis are summarized for both the solids profiles (left hand side) and the catalytic performance profiles (right hand side) by plotting the similarity measure versus similarity rank. Hereby, each of the four catalyst samples was chosen ones as master to whom the similarity of the residual three catalysts was referred. The symbol of the "master" catalyst is located in the upper left corner of each figure (highest similarity value (= 1) and lowest rank with respect to similarity (= 1)).

It can be seen that there is no complete agreement in the plots for solids' characteristics profiles and catalytic performance profiles since the similarity with respect to solids' characteristics between DEG-cat 1 and DEG-cat 3 is close while it is not for the catalytic performance profiles. From this finding it can be concluded that some of the test reactions are probably influenced by solid properties which have yet not been covered by the solids parameters.

Thus, catalytic profiling is a more sensitive indicator for modifications of preparation methods than profiling of physical properties. Catalytic profiling is an efficient and effective method for optimisation and development of catalysts for fine-chemical application.

### *Example 2*

The example demonstrates that a data base comprising activity data from hydrogenation of mono-functional substrates allows a pre-selection of potential catalysts for hydrogenation of multifunctional substrates. Based on this pre-selection concept the process of identifying the optimal precious metal powder catalysts is accelerated.

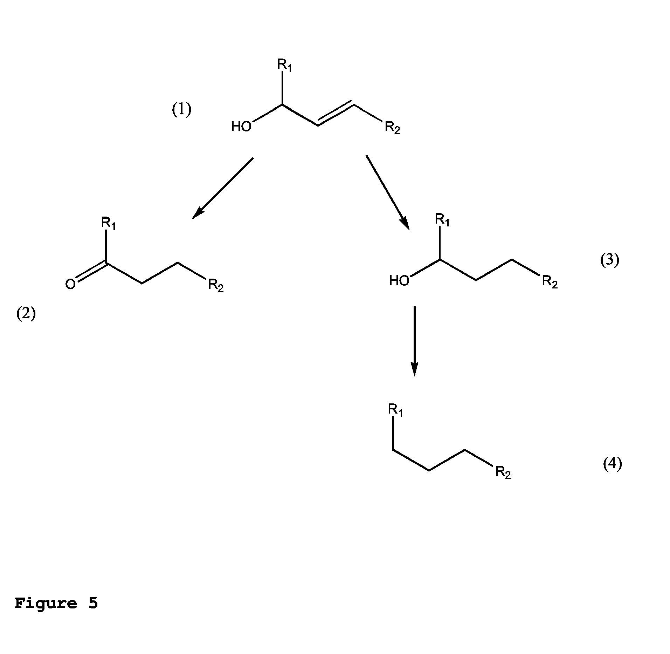

A catalyst which leads to selective formation of saturated alcohol according to reaction scheme in Figure 5 shall be identified among a group of sixteen different Pd-catalysts prepared by different methods and showing different metal particle size, metal dispersion and oxidation state of Pd. For pre-selection of promising catalysts, profiling data concerning C=C double bond hydrogenation and hydrogenolysis are of interest.

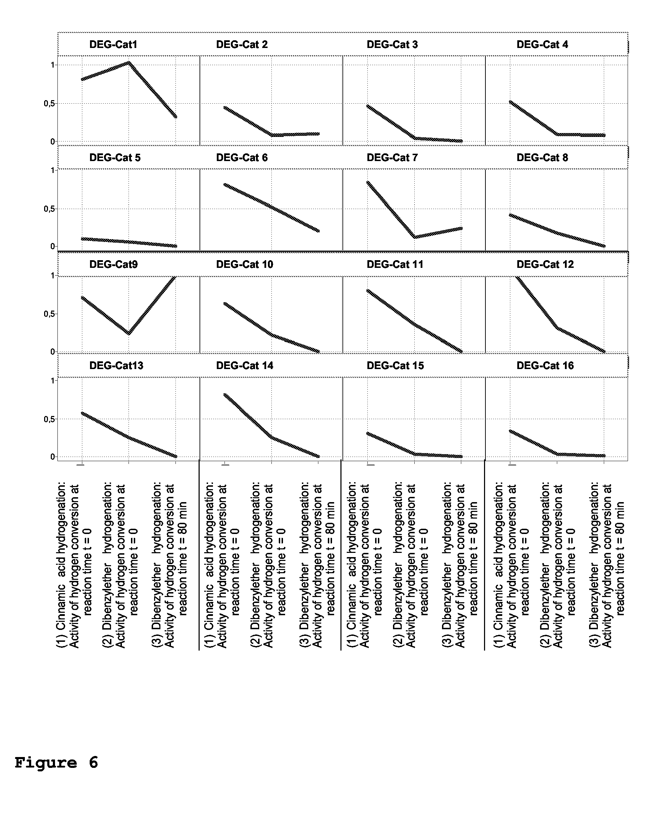

Activity data for hydrogenation of cinnamic acid which represents C=C double bond hydrogenation and debenzylation of debenzylether which represents hydrogenolysis were chosen as pre-selection criteria and visualized by performance profiles (Fig. 6). The profiles refer to the following activity values:

*Hydrogenation of cinnamic acid at 10 bars and 25°C*
(1) Activity of hydrogen conversion at reaction time t = 0

*Hydrogenation of dibenzylether at 10 bars and 25°C*
(2) Activity for hydrogen conversion at reaction time t = 0
(3) Activity for hydrogen conversion at reaction t = 80 min

For the activity tests a eightfold batch reactor system (reactor volume 20 ml) with magnetic stirring which allows the measurement of hydrogen uptake at constant hydrogen pressure was used. Analysis of substrates and products was performed off-line by gaschromatography for determining selectivity values. Activity values were derived from hydrogen up-take within a defined time interval.

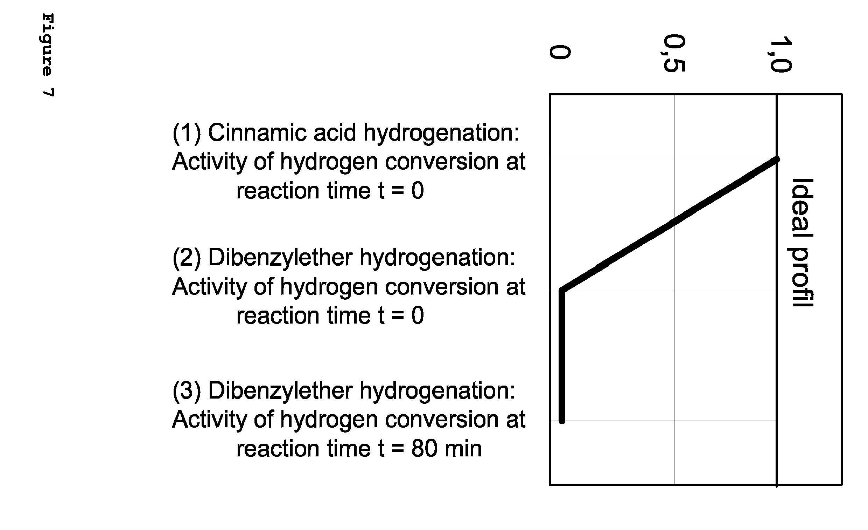

Catalysts which are expected to be highly selective in the hydrogenation of hydroxy-olefin (Figure 5) should reveal high activity in C=C-double bond hydrogenation but low activity in hydrogenolysis. Accordingly, a hypothetical performance profile can be drawn which reflects an ideal catalyst revealing highest activity in C=C-double bond hydrogenation and zero activity in the hydrogenolysis as shown in Figure 7. Now, the profiles of the real catalysts shown in Fig. 6 can be compared with the hypothetical ideal profile based on statistical similarity analysis. Those of the sixteen different Pd catalysts in Fig. 6 which are most similar to the hypothetical profile should correspond to the preferable catalysts for selective hydrogenation of the hydroxy-olefin shown in Figure 5.

The statistical similarity analysis was performed based on determination of Euclidean distance between hypothetical and catalyst profile according to the following formula:

$$similarity = \sqrt{\frac{(real\ perfomance\ value - ideal\ performance\ value)^2}{(ideal\ performance\ value)^2}}$$

Since relative distances are considered in this formula small positive deviations from zero-activity for the hydrogenolysis are strongly weighted.

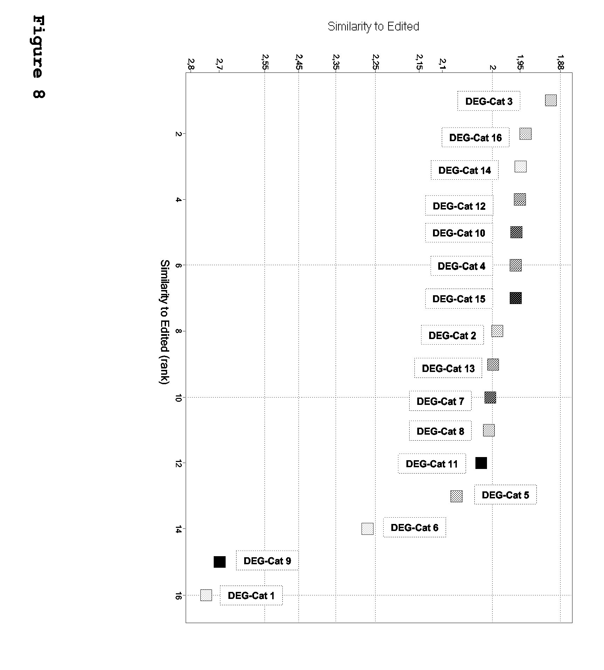

Figure 8 indicates the ranking of similarity of the sixteen Pd catalysts with respect to the hypothetical ideal profile shown in Figure 7. Accordingly, catalyst DEG-3 appears to be the preferable catalyst for the selective hydrogenation of hydroxy-olefin. DEG-16, DEG-14 and DEG-12 are expected to give also high yield of the saturated alcohol.

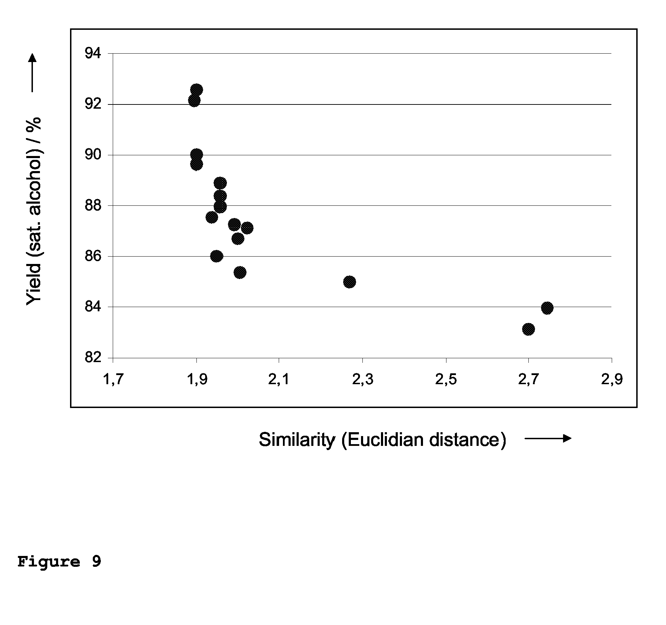

The proof that this pre-selection meets indeed the most selective catalysts for the hydrogenation of the hydroxy-olefin is derived from Figure 9. There, the yield of saturated alcohol obtained by conversion of the 1-hydroxy-3,4-olefin (see Figure 5) is plotted versus the similarity values derived from the Euclidean distance between performance profiles of real catalysts (Figure 6) and the ideal profile (Figure 7). The correlation between yield and similarity is significant. Therefore, the catalytic performance profiling appears to be a fast and unerring method for pre-selection of catalysts for hydrogenation of multi-functional substrates.

*Example 3*

This example demonstrates that the profiling method allows a straightforward optimisation of a catalyst preparation method with respect to preparation recipe and, hence, production costs.

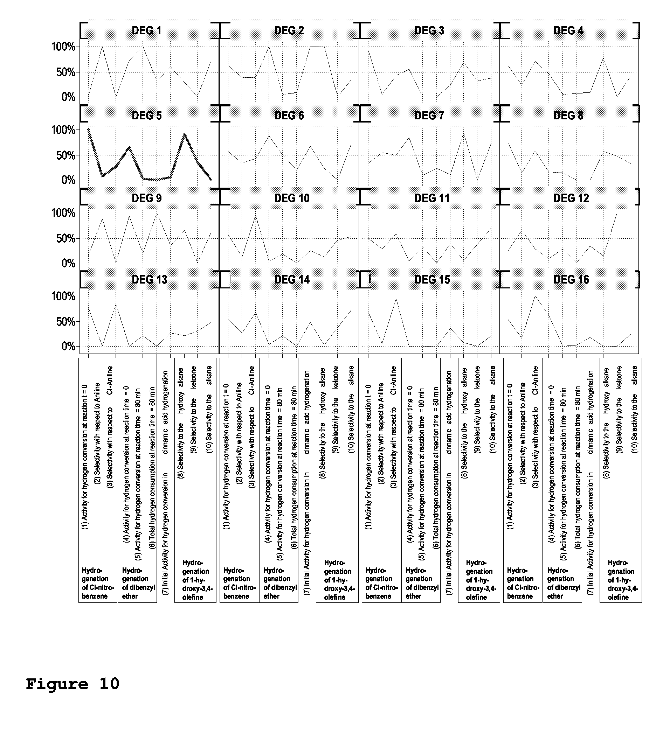

A catalyst DEG 5 (see Fig. 10) shows excellent and unique catalytic performance for selective hydrogenation of multiple C=C double bonds and selective hydrogenation of Cl-nitro-aromatics. Therefore, it is the preferred catalysts for hydrogenation of multi-functional substrates comprising Cl-substituted nitro-aromatics as well as C=C double bonds. The preparation of this catalyst, however, is costly. Fifteen alternative modifications of the DEG-5 preparation method were developed with the aim to maintain the complete catalytic performance profile of the DEG-5 catalyst but to minimize the catalyst production effort. Catalytic performance profiles of theses samples indicated in Fig. 10 refer to the chemical reactions mentioned in Example 1.

For the experimental tests a eightfold batch reactor system (reactor volume 20 ml) with magnetic stirring which allows the measurement of hydrogen uptake at constant hydrogen pressure was used. Analysis of substrates and products was performed off-line by gaschromatography for determining selectivity values. Activity values were derived from hydrogen up-take within a defined time interval.

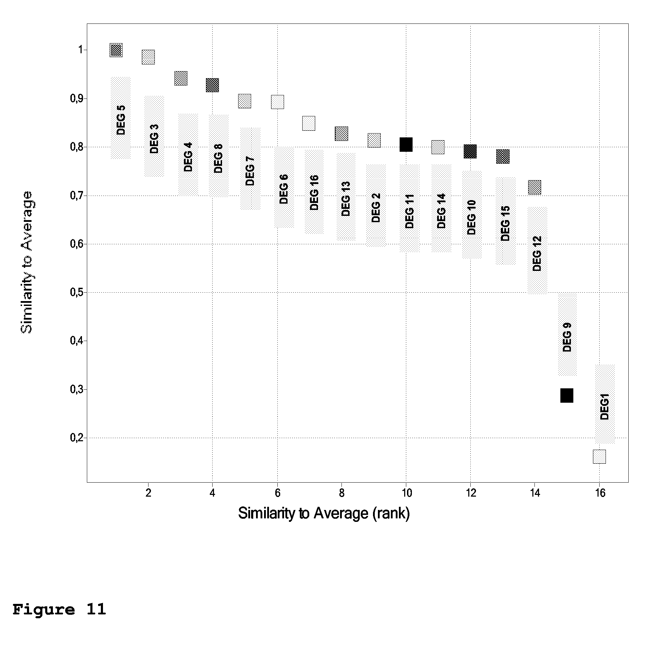

Based on similarity analysis those alternative samples were identified which where approximated to DEG-5 with respect to the catalytic performance profile (Fig. 11).

In this example this is fulfilled by DEG-3. This catalyst was prepared based on a much simpler recipe related to lower expenses for raw material (especially reducing agent) and to saving of production time.

**Claims**

1.   Catalyst, **characterised in that**, it is defined by a specific performance profile.

2.   Catalyst according to claim 1, **characterised in that**, the catalyst is a heterogeneous catalyst.

3.   Method to define the specific performance profile of the catalyst according to claim 1 or 2, **characterised in that**, the catalyst is synthezide and then is used in at least two different chemical reactions assigned to various chemical reaction classes comprising preferentially
     hydrogenation of carbonyl compounds
     hydrogenation of olefins or polyolefins
     hydrogenation of aromatics or heteroaromatics
     hydrogenation of nitro-compounds
     hydrogenation of nitriles,
     hydrogenation of imines,
     hydrogenation of hydroxylamines,
     hydrogenation of alkynes,
     reductive alkylation of primary or secondary amines,
     reductive amination of aldehydes or ketones by ammonia salts or by amines,
     hydrogenolysis of C-C bonds, ethers, carbamates, carbonates, amines or organic sulfides,
     hydrodehalogenation of halo-aromatics or halo-aliphatics,
     dehydrogenation of cycloalkanes or cycloalkenes, isomerization of hydroxy-olefins,
     hydrogenation of multifunctional substrates including at least two of the following functional groups or structural units: CC-double bond CC-triple bond, nitro-, alcohol-, carbonyl-, carboxyl- , nitril-, imine, hydroxylamine-, azo-, diazo-, halogen-, ether-group, aromatic rings,
     oxidation of alcoholes,
     oxidation of aldehydes,
     oxidation of olefins,
     oxidation of multifunctional substrates including at least two of the following functional groups: CC-double bond, CC triple bond, alcohol-, carbonyl -, nitril-, imine, hydroxylamine-, azo-, diazo-group,
     C-C coupling,
     enantioselective hydrogenation of carbonyl compounds,
     enantioselective reductive alkylation of primary or secondary amines,
     enantioselective reductive amination of aldehydes or ketones by ammonia salts or by amines,
     whereby in respect to each reaction the catalyst performance is estimated and correlated to a defined table in order to set up the performance profile.

4.   Library of catalysts **characterised in, that** each catalyst is defined by a specific performance profile.

5.   Library of catalysts according to claim 4, **characterised in that**, the library can be searched using an algorithm of the statistical similarity analysis.

6.   Library according to claim 4 or 5, **characterised in, that** the catalysts are heterogeneous catalysts.

7.   Method to produce the library of catalysts according to claim 1, **characterised in, that** each catalyst is synthesized separately and then is used in at least two different chemical reactions assigned to various chemical reaction classes comprising preferentially

hydrogenation of carbonyl compounds

hydrogenation of olefins or polyolefins

hydrogenation of aromatics or heteroaromatics

hydrogenation of nitro-compounds

hydrogenation of nitriles,

hydrogenation of imines,

hydrogenation of hydroxylamines,

hydrogenation of alkynes,

reductive alkylation of primary or secondary amines,

reductive amination of aldehydes or ketones by ammonia salts or by amines,

hydrogenolysis of C-C bonds, ethers, carbamates, carbonates, amines or organic sulfides,

hydrodehalogenation of halo-aromatics or halo-aliphatics,

dehydrogenation of cycloalkanes or cycloalkenes, isomerization of hydroxy-olefins,

hydrogenation of multifunctional substrates including at least two of the following functional groups or structural units: CC-double bond CC-triple bond, nitro-, alcohol-, carbonyl-, carboxyl- , nitril-, imine, hydroxylamine-, azo-, diazo-, halogen-, ether-group, aromatic rings,

oxidation of alcoholes,

oxidation of aldehydes,

oxidation of olefins,

oxidation of multifunctional substrates including at least two of the following functional groups: CC-double bond, CC triple bond, alcohol-, carbonyl- , nitril-, imine, hydroxylamine-, azo-, diazo-group,

C-C coupling,

enantioselective hydrogenation of carbonyl compounds,

enantioselective reductive alkylation of primary or secondary amines,

enantioselective reductive amination of aldehydes or ketones by ammonia salts or by amines,

whereby in respect to each reaction the catalyst performance is estimated and correlated to a defined table in order to set up the performance profile, further on the catalyst is put into the library.

8. Method to elect a catalyst from the library of catalysts according to claim 1 in respect to a given substrate, **characterised in, that** the substrate, which should be treated with the catalyst, can produce a plurality of compounds, whereby one specific compound is wanted to be produced selectively, whereby the substrate shows a specific profile in respect to the performance of the catalyst needed and this performance profile is compared to the performance profiled of the catalyst of the library according to claim 1.

9. Method according to claim 5, **characterised in, that** the election of the catalyst is performed by using an algorithm of the statistical similarity analysis.

a)

b)

**Figure 1:** Illustration of the concept for catalyst profiling based on a set of sensitive test reactions:

> a) Determination of particular performance data for an individual catalyst (activity and selectivity values in different test reactions)
> b) Visualization of performance profiles from the particular performance values as fingerprints for individual catalysts as a basis of similarity analysis

**Figure 2:**

Catalytic performance profiles of four Pd(5-wt%)/C catalysts (identical activated carbon support)

DEG-cat 1 | DEG-cat 2

100%
50%
0%

DEG-cat 3 | DEG-cat 4

100%
50%
0%

(1) asymmetry of particle size distribution referring to metal particle number
(2) asymmetry of particle size distribution referring to metal particle volume
(3) inconsistency of particle size distribution referring to metal particle number
(4) inconsistency of particle size distribution referring to metal particle volume
(5) kurtosis of particle size distribution referring to metal particle number
(6) kurtosis of particle size distribution referring to metal particle volume
(7) mean volume of metal particles / nm³
(8) metal particle size distribution referring to metal particle number / nm
(9) particle size distribution referring to metal particle volume e / nm
(10) relative standard deviation of particle size distribution referring to particle number /%
(11) relative standard deviation of metal particle size distribution referring to volume / %
(12) specific surface area of metal particles / m² cm⁻³
(13) standard deviation of particle size distribution referring to particle number / nm
(14) standard deviation of particle size distribution referring to particle volume / nm
(15) XPS Binding energy derived from the Pd 3d 5/2 signal
(16) XPS Binding energy of derived from the O 1s signal
(17) surface atom fraction of palladium (derived from XPS analysis)
(18) surface atom fraction of oxygen (derived from XPS analysis)

(1) asymmetry of particle size distribution referring to metal particle number
(2) asymmetry of particle size distribution referring to metal particle volume
(3) inconsistency of particle size distribution referring to metal particle number
(4) inconsistency of particle size distribution referring to metal particle volume
(5) kurtosis of particle size distribution referring to metal particle number
(6) kurtosis of particle size distribution referring to metal particle volume
(7) mean volume of metal particles / nm³
(8) metal particle size distribution referring to metal particle number / nm
(9) particle size distribution referring to metal particle volume e / nm
(10) relative standard deviation of particle size distribution referring to particle number /%
(11) relative standard deviation of metal particle size distribution referring to volume / %
(12) specific surface area of metal particles / m² cm⁻³
(13) standard deviation of particle size distribution referring to particle number / nm
(14) standard deviation of particle size distribution referring to particle volume / nm
(15) XPS Binding energy derived from the Pd 3d 5/2 signal
(16) XPS Binding energy of derived from the O 1s signal
(17) surface atom fraction of palladium (derived from XPS analysis)
(18) surface atom fraction of oxygen (derived from XPS analysis)

**Figure 3:**

Solids' characteristics profiles (identical activated carbon support)

**Similarity plots**

for characterization profiles                    for catalytic performance profiles

### Similarity referred to DEG-cat 1

### Similarity referred to DEG-cat 2

### Similarity referred to DEG-cat 3

### Similarity referred to DEG-cat 4

**Figure 4**

Figure 5

Figure 6

Figure 7

Figure 8

Figure 9

Figure 10

Figure 11

European Patent
Office

**PARTIAL EUROPEAN SEARCH REPORT**

Application Number

which under Rule 45 of the European Patent Convention
shall be considered, for the purposes of subsequent
proceedings, as the European search report

EP 06 11 0693

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 3 584 039 A (DELBERT H. MEYER) 8 June 1971 (1971-06-08) * column 5, line 20 - line 24; table 1 * * column 4, line 34 - line 50 * ----- | 2 | INV. B01J19/00 G01N31/10 C40B40/18 |
| X | US 2004/014224 A1 (LOPEZ NORBERT) 22 January 2004 (2004-01-22) * abstract * * paragraph [0019] * * paragraph [0098] - paragraph [0106]; claims; figure 1 * ----- | 2,3,6-9 | |
| X | DE 101 01 118 A1 (INSTITUT FUER ANGEWANDTE CHEMIE BERLIN-ADLERSHOF E.V) 18 July 2002 (2002-07-18) * abstract * * paragraph [0006] - paragraph [0017]; figures; example * ----- -/-- | 2,3,6-9 | |

TECHNICAL FIELDS
SEARCHED        (IPC)

B01J
G01N
C40B

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 7 August 2006 | Nazario, L |

EPO FORM 1503 03.82 (P04C07)

European Patent
Office

**PARTIAL EUROPEAN SEARCH REPORT**

Application Number

EP 06 11 0693

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| X | US 2003/078740 A1 (KIEKEN LAURENT ET AL) 24 April 2003 (2003-04-24) * abstract * * paragraph [0037] - paragraph [0044] * * paragraph [0053] - paragraph [0077] * * claims; figures 1-3; tables 1,2 * ----- | 2,3,6-9 | |
| X | US 2002/042140 A1 (HAGEMEYER ALFRED ET AL) 11 April 2002 (2002-04-11) * abstract * * paragraph [0006] * * paragraph [0008] * * paragraph [0011] * * paragraph [0015] * * paragraph [0026] * * paragraph [0032] * * paragraph [0075] * * paragraph [0077] * * paragraph [0079] * * paragraph [0094] - paragraph [0110] * * paragraph [0127] - paragraph [0136]; claims; figure 5 * ----- | 2,3,6-9 | TECHNICAL FIELDS SEARCHED (IPC) |

EPO FORM 1503 03.82 (P04C10)

European Patent
Office

INCOMPLETE SEARCH
SHEET C

Application Number

EP 06 11 0693

Claim(s) not searched:
     1, 4, 5

Reason for the limitation of the search:

Present claim 1 relates to a catalyst, however no technical features
(e.g. composition, structure, size, porositiy, ...) of the said catalyst
are disclosed. The claim merely discloses that the catalyst is defined
by a specific performance profile. Such a formulation attempts to define
the catalyst by way of how well (or how badly) it performs in an
unspecified use and does not characterise the catalyst itself and is
therefore unclear (Article 84 EPC). Given that there are no technical
features defining the catalyst, any chemical species capable of acting as
a catalyst would antecipate the subject-matter of the present claim.

A similar objection applies to claims 4 and 5. These claims relate to
libraries of catalysts, however no technical features characterising the
claimed subject-matter are disclosed. Claim 4 merely discloses that each
catalyst is defined by a specific performance profile. Such a
formulation attempts to define the catalyst by way of how well (or how
badly) it performs in an unspecified use and does not characterise the
catalyst itself and is therefore unclear (Article 84 EPC). Likewise,
claim 5 attempts to define the library by of its use, i.e. a library that
can be searched using mathematical tools, but does not further
characterise the library itself and is unclear. Given that there are no
technical features defining the catalyst library, any library of
chemical species capable of acting as catalysts would antecipate the
subject-matter of the present claim.

For these reasons, a meaningful search of the claimed subject matter of
claims 1, 4 and 5 could not be carried out (Rule 45 EPC).

## ANNEX TO THE EUROPEAN SEARCH REPORT
## ON EUROPEAN PATENT APPLICATION NO.

EP 06 11 0693

This annex lists the patent family members relating to the patent documents cited in the above-mentioned European search report.
The members are as contained in the European Patent Office EDP file on
The European Patent Office is in no way liable for these particulars which are merely given for the purpose of information.

07-08-2006

| Patent document cited in search report | | Publication date | Patent family member(s) | | Publication date |
|---|---|---|---|---|---|
| US 3584039 | A | 08-06-1971 | NONE | | |
| US 2004014224 | A1 | 22-01-2004 | AT | 258541 T | 15-02-2004 |
| | | | AU | 7431100 A | 24-04-2001 |
| | | | BR | 0014077 A | 14-05-2002 |
| | | | CA | 2384703 A1 | 29-03-2001 |
| | | | CN | 1391541 A | 15-01-2003 |
| | | | DE | 60008018 D1 | 04-03-2004 |
| | | | DE | 60008018 T2 | 11-11-2004 |
| | | | EP | 1232131 A1 | 21-08-2002 |
| | | | ES | 2215068 T3 | 01-10-2004 |
| | | | FR | 2798659 A1 | 23-03-2001 |
| | | | WO | 0121560 A1 | 29-03-2001 |
| | | | JP | 2003509580 T | 11-03-2003 |
| | | | MX | PA02002807 A | 23-10-2002 |
| | | | PT | 1232131 T | 31-05-2004 |
| DE 10101118 | A1 | 18-07-2002 | WO | 02053287 A2 | 11-07-2002 |
| US 2003078740 | A1 | 24-04-2003 | WO | 03020417 A1 | 13-03-2003 |
| US 2002042140 | A1 | 11-04-2002 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HAIR, J.F.JR. ; ANDERSON, R.E. ; TATHAM, R.L. ; BLACK, W.C.** Multi-variate Data Analysis. Prentice Hall, 1995